# EUROPEAN PATENT SPECIFICATION

(11) **EP 3 505 144 B1**
(45) Date of publication and mention of the grant of the patent: **01.05.2024**
(21) Application number: 19157571.1
(22) Date of filing: 03.10.2014
(51) Int. Cl.: A61F 7/12, A61M 25/01, A61F 7/00, F28D 9/00, A61F 7/02, F28F 3/12, F25B 39/02

(54) **PATIENT HEAT EXCHANGE SYSTEM**
PATIENTENWÄRMEAUSTAUSCHSYSTEM
SYSTÈME D'ÉCHANGE THERMIQUE DE PATIENT

(30) Priority: 07.02.2014 US 201414175545; 13.05.2014 US 201414276202
(43) Date of publication of application: 03.07.2019
(62) Divisional of application: 14881612.7
(73) Proprietor: ZOLL Circulation, Inc., San Jose, CA 95131 (US)
(72) Inventor: DABROWIAK, Jeremy Thomas, Redwood City, CA 94065 (US); PETERSON, Eric, Pacifica, CA 94044 (US)
(74) Representative: Potter Clarkson

(56) References cited:
- WO-A2-91/10414
- US-A- 4 707 587
- US-A- 4 731 072
- US-A1- 2004 026 068
- US-A1- 2004 175 165
- US-A1- 2008 262 409
- US-A1- 2008 269 676
- US-A1- 2011 046 551
- US-B1- 6 878 156

## Description

### II. BACKGROUND OF THE INVENTION

Patient temperature control systems have been introduced to prevent fever in patients in the neuro ICO due to suffering from sub-arachnoid hemorrhage or other neurologic malady such as stroke. Also, such systems have been used to induce mild or moderate hypothermia to improve the outcomes of patients suffering from such maladies as stroke, cardiac arrest, myocardial infarction, traumatic brain injury, and high intracranial pressure. Examples of intravascular heat exchange catheter are disclosed in U.S. Patent Nos. 6,419,643, 6,416,533, 6,409,747, 6,405,080, 6,393,320, 6,368,304, 6,338,727, 6,299,599, 6,290,717, 6,287,326, 6,165,207, 6,149,670, 6,146,411, 6,126,684, 6306,161, 6,264,679, 6,231,594, 6,149,676, 6,149,673, 6,110,168, 5,989,238, 5,879,329, 5,837,003, 6,383,210, 6,379,378, 6,364,899, 6,325,818, 6,312,452, 6,261,312, 6,254,626, 6,251,130, 6,251,129, 6,245,095, 6,238,428, 6,235,048, 6,231,595, 6,224,624, 6,149,677, 6,096,068, 6,042,559.

External, patient temperature control systems may be used. Such systems are disclosed in U.S. Patent Nos. 6,827,728, 6,818,012, 6,802,855, 6,799,063, 6,764,391, 6,692,518, 6,669,715, 6,660,027, 6,648,905, 6,645,232, 6,620,187, 6,461,379, 6,375,674, 6,197,045, and 6,188,930 (collectively, "the external pad patents"). US 2011/046551 describes a fluid warming cassette system in which the cassette has a stiffening frame structure. The fluid container is thin to minimize heat exchange inefficiencies. The frame structure permits the thin fluid container to be inserted into a narrow space between fixed position warming plates of a warming unit. The frame structure has a quadrilateral shape with sides and ends. The fluid container is attached, at its periphery to the sides and ends of the frame structure, within the quadrilateral shape. The fluid container is made of two sheets of thermally conductive plastic film material bonded together in a serpentine pattern which creates a fluid channel between the sheets. US 2004/0026068 describes a fluid warming cassette with a first sheet and second sheet joined with a flexed tensioning rod to form a fluid container with a periphery in which the flexed tensioning rod is disposed to tension the fluid container. The sheets of plastic film material are bonded in a pattern which creates a serpentine fluid channel between the sheets. US 2008/0262409 describes a heat exchanger comprising a casing with a serpentine pathway, a membrane enclosed by the casing, an inlet tube for fluid to enter the heat exchanger and an outlet tube for fluid to exit the heat exchanger. US 2004/0175165 describes a fluid warming cassette having two polymeric films and two guide rails and a tortuous pattern for a fluid path section. US 4731072 describes an apparatus for heating or cooling fluids having a container in the form of a generally flat bag whose two major walls comprise relatively thin, generally flexible sheets of plastic material secured together and centrally located compartment having means for directing flow of liquid back and forth within the compartment. US 2008/0269676 describes a heat exchanger with a fluid flow path receivable between heating plates.

### SUMMARY OF THE INVENTION

The invention provides a device according to claim 1. More generally, aspects of the present disclosure include a heat exchange system for exchanging heat with working fluid from an intravascular heat exchange catheter or an external heat exchange pad includes a refrigerant circuit configured for circulating refrigerant between a compressor and first and second cold plates between which a working fluid cassette is disposable.

In example embodiments a cassette slot is defined between the cold plates for receiving the fluid cassette. A distance between the cold plates (e.g., the width of the slot) can be less than forty mils (0.040") (1.02 mm). Plural fasteners may hold the outer wall onto metal structure of the first cold plate. The fasteners may be arranged in four rows, one respective row along each of four edges of the outer wall. The cold plates can be nearly square and can abut each other along left and right side walls. In examples, respective vertically elongated cassette frame receptacles are located immediately inboard of the respective side walls with the slot extending between the side walls and terminating at the receptacles, and the frame receptacles are wider than the slot. At least one cold plate may be formed with a serpentine passageway through which the refrigerant flows.

In another aspect, system includes two heat transfer plates parallel to each other and defining a slot between them configured for receiving a working fluid cassette through which working fluid flows to and from an intravascular catheter in a working fluid circuit. A refrigerant circuit supplies refrigerant to at least one of the plates to exchange heat therewith. The refrigerant circuit includes a compressor and may be the only fluid circuit in thermal contact with the working fluid circuit other than a bloodstream of a patient in which the catheter can be positioned. According to present principles, at least one of the heat transfer plates is at least partially transparent to permit viewing the refrigerant as it flows in the refrigerant circuit.

In another aspect, a method includes circulating refrigerant between a compressor and a cold plate, and enabling a person to view the refrigerant as it circulates.

The details of the present invention, both as to its structure and operation, can. best be understood in reference to the accompanying drawings, in which like reference numerals refer to like parts, and in which:

### BRIEF DESCRIPTION OF THE DRAWINGS

Figure 1 is a schematic view of a heat exchange system;
Figure 2 is a perspective view of an example working fluid cassette holder portion of a heat exchange system;
Figure 3 is another view of the transparent half of the cassette holder shown in Figure 2 omitting the fasteners and holes for clarity;
Figure 4 is a perspective view of an example working fluid cassette configured to engage the cassette holder shown in Figures 2 and 3; and
Figure 5 is a close up perspective view of the cassette shown in Figure 4, illustrating an inlet tube extending partially down into the stretched membrane chamber, it being understood that an opposed outlet tube may be similarly disposed on the opposite side of the cartridge and that both the inlet and outlet tubes may extend any length down their respective sides in the cassette.

### DETAILED DESCRIPTION OF THE PREFERRED EMBODIMENT

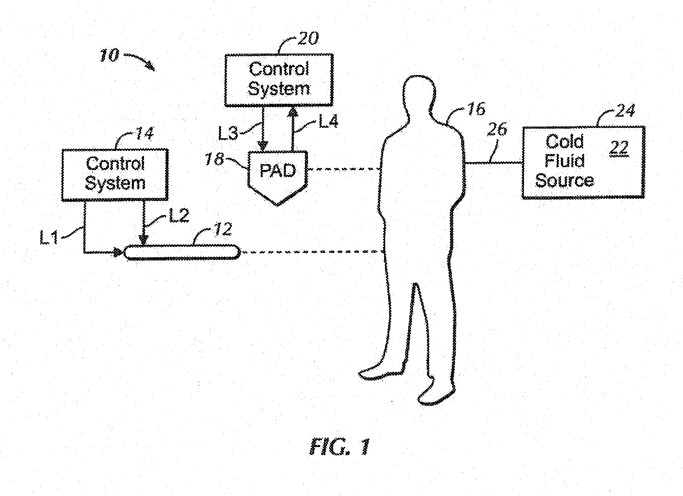

Referring initially to Figure 1, in accordance with present principles, a system 10 may include an intravascular heat exchange catheter 12 controlled by a control system 14 to induce control patient temperature, e.g., to prevent the patient 16 from becoming febrile or to induce therapeutic hypothermia in the patient 16. In the catheter, working fluid (also referred to as "coolant") such as but not limited to saline circulates (typically under the influence of a pump in the controller) in a dosed loop from the control system 14, through a fluid supply line L1, through the catheter 12, and back to the system 14 through a fluid return line L2, such that no coolant enters the body. While certain preferred catheters are disclosed below, it is to be understood that other catheters can be used in accordance with present principles, including, without limitation, any of the catheters disclosed above or the following U.S. patents: USPN 5,486,208, 5,837,003, 6,110,168, 6,149,673, 6,149,676, 6,231,594, 6,264,679, 6,306,161, 6,235,048, 6,238,428, 6,245,095, 6,251,129, 6,251,130, 6,254,626, 6,261,312, 6,312,452, 6,325,818, 6.409,747, 6,368,304, 6,338,727, 6,299,599, 6,287,326, 6,126,684. The catheter 12 may be placed in the venous system, e.g., in the superior or inferior vena cava,

Instead of or in addition to the catheter 12, the system 10 may include one or more pads 18 mat are positioned against the external skin of the patient 16 (only one pad 18 shown for clarity). The pad 18 may be, without limitation, any one of the pads disclosed in the external pad patents. The temperature of the pad 18 can be controlled by a pad control system 20 in accordance with principles set forth in the external pad patents to exchange heat with the patient 16, including to induce therapeutic mild or moderate hypothermia in the patient in response to the patient presenting with, e.g., cardiac arrest, myocardial infarction, stroke, high intracranial pressure, traumatic brain injury, or other malady the effects of which can be ameliorated by hypothermia. The pad 18 may receive working fluid from the system 20 through a fluid supply line L3, and return working fluid to the system 20 through a fluid return line L4. Note that in some embodiments, the systems 14, 20 are established in a single assembly.

To cool the patient while awaiting engagement of the catheter 12 and/or pad 18 with the patient, cold fluid 22 in a cold fluid source 24 may be injected into the patient and in particular into the patient's venous system through a pathway 26. Without limitation, the pathway 26 may an IV line, the source 24 may be an IV bag, and the fluid 22 may be chilled saline, e.g., saline at the freezing point or slightly warmer. Or, the source may be a syringe, and the saline can be injected directly into the bloodstream of the patient.

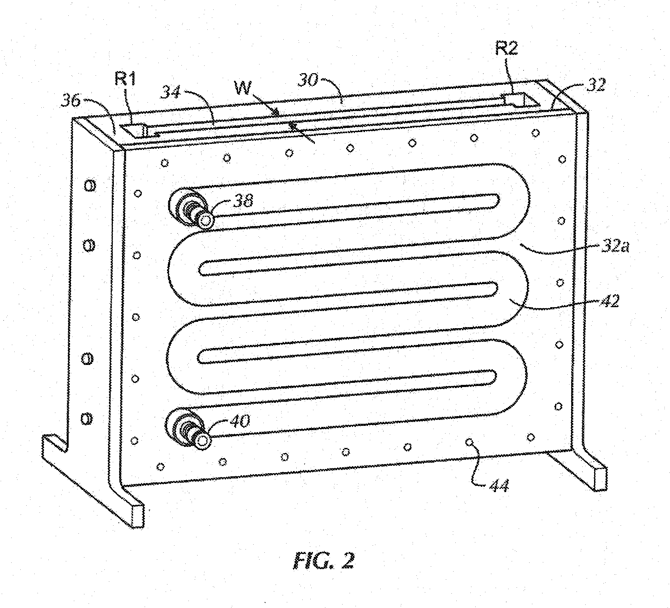

Now referring to Figure 2, a portion of either of the heat exchangers in the control systems 14, 20 is shown which includes at least two cold plates 30, 32 defining a cassette slot 34 between them, in one embodiment the width "W" of the slot 34 is less than forty mils (0.040") (1.02 mm), and may be between twenty nine mils and thirty one mils (0.029"-0.031") (0.74 - 0.79 mm) or may have a nominal slot width of 0.035" (0.89 mm). In a specific example the width "W" may be thirty mils (0.76 mm). In other embodiments, when a disposable heat exchange bag with serpentine channels is used, a larger gap between the cold plates may be used, e.g., 0.060" - 0.120" (1.52 - 3.05 mm) and more preferably 0.080" (2.03 mm) to promote pumping saline through the bag without excessive backpressure.

The cold plates 30, 32 may be made of metal with the exception noted below, and can be rectilinear as shown and indeed may be nearly square. The cold plates 30, 32 may abut each other along left and right side walls 36, with elongated vertical cassette frame receptacles R1 and R2 being located immediately inboard of the respective side walls 36 and with the slot 34 extending between the walls 36 and terminating at the receptacles R1, R2 as shown. The frame receptacles R1, R2 are wider than the slot 36.

In the example shown, refrigerant inlet and outlet tubes 38, 40 extend through at least one of the cold plates 32 to communicate refrigerant from a compressor into a refrigerant passageway in the cold plate. Each cold plate may have its own refrigerant inlet and outlet tubes, or, in the embodiment shown, only one cold plate may be formed with refrigerant inlet and outlet tubes and the other cold plate either thermally coupled to the cold plate in which the refrigerant flows and/or receiving refrigerant from the other cold plate through passageways formed through one or both of the side walls 36.

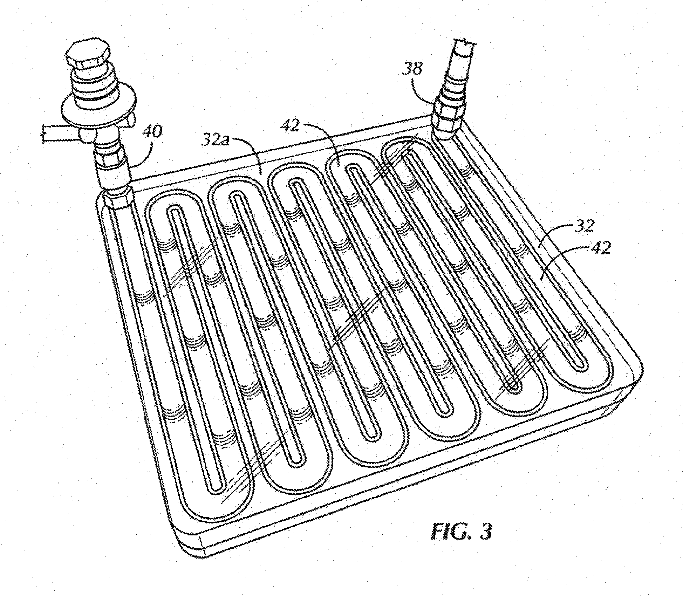

In the example shown in Figures 2 and 3, the outer wall of at least one of the cold plates is made at least partially (in the example shown, the entire wall is transparent) of transparent material such as hard plastic, e.g., plexiglass having a thickness of, e.g., one eighth of an inch (3.18 mm), so that a person can view refrigerant flowing inside the cold plate to verify proper operation. For example, the outer wall 32a of the cold plate 32 may be transparent as shown.

Thus, as shown in Figures 2 and 3, owing to the transparent outer wall 32a of the cold plate 32, the refrigerant, in this non-limiting example constrained to flow in a serpentine refrigerant passageway 42, can be viewed during operation by a person looking at the outer wall 32a. The example refrigerant passageway that fluidly connects the refrigerant inlet 38 to the refrigerant outlet 40 may be serpentine-shaped as shown, or may be some other shape or pattern such as a herringbone pattern, a wave pattern, etc. Alternatively, parallel channel passages may be used. For instance, ten one-inch (25.4 mm) wide channels may be formed in parallel, thereby achieving a 10x10" (25.4 x 25.4 cm) surface area. Typically during operation the refrigerant bubbles as it transitions state while exchanging heat with the cassette and so the visual presentation to the viewer is of a flowing bubbling fluid.

Because the transparent outer wall 32a is exposed to fluid pressure from the inside, plural fasteners 44 (Figure 2) may be used to secure the outer wall 32a to the remaining metal structure of the cold plate 32. Note that Figure 3 does not show the fasteners for clarity, to better reveal the transparent view of the underlying refrigerant path.

The fasteners 44 shown in Figure 2 may be threaded fasteners such as bolts that are received in corresponding threaded receptacles with which they are registered in the metal structure of the cold plate 32. For fortified securement, Figure 2 shows that four rows of fasteners 44 may be used, a respective row of fasteners being arranged along each of the four edges of the wall 32a just inboard of the periphery of the wall. The spacing between adjacent fasteners may be, e.g., one half of an inch (12.7 mm).

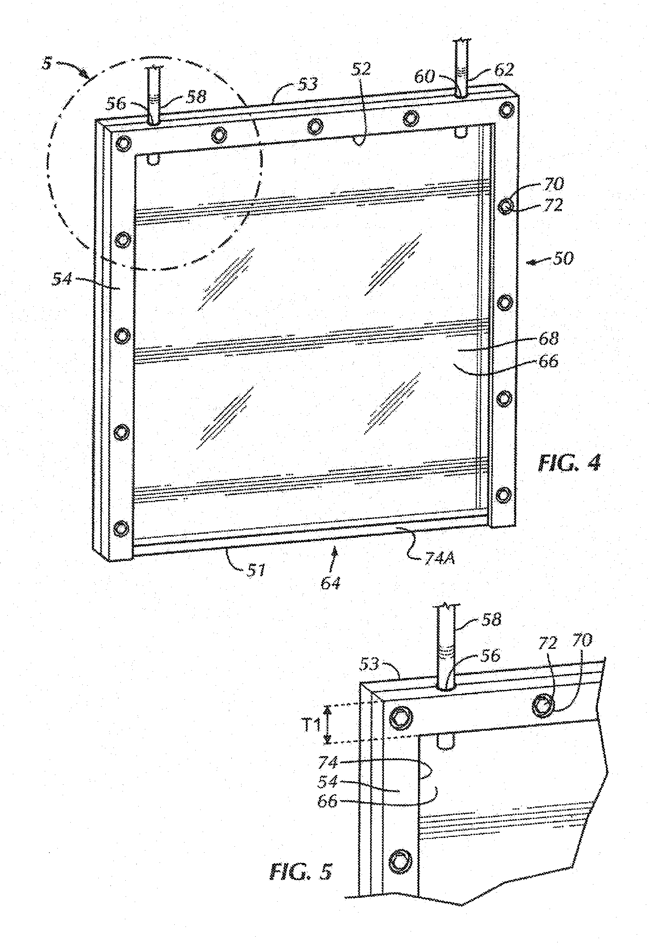

Figure 4 shows an example working fluid cassette 50 according to present principles. The cassette 50 is configured to fit snugly into the slot 34 and cassette frame receptacles R1, R2 defined between the cold plates 30, 32. Working fluid such as saline from a patient-engageable heat exchange member such as the catheter 12 or pad 18 flows through the cassette 50 in operation, with the working fluid exchanging heat with the refrigerant in the cold plates. In example embodiments, the cassette 50 is a low cost single-use disposable item that can contain, e.g., sterile saline which circulates through the catheter 12. The cassette may be placed by a medical caregiver in the slot 34 between the cold plates 30, 32 and the membrane portion which defines a space or working fluid chamber through which the example saline flows inflates when the working fluid flows through it, achieving thermal contact with the cold plates 30, 32,

In the example shown, the cassette 50 includes a frame 52 defining a periphery and a preferably rectilinear opening bounded as shown on at least three sides by the periphery of the frame. In the non-limiting example shown, the frame includes an elongated parallelepiped-shaped top rail 53 and elongated parallelepiped-shaped left and right side rails 54 parallel to each other and perpendicular to the top rail 32. The example frame 52 has no bottom rail opposite the top rail. In any case, the example frame 52 is rectilinear and is configured for being closely received between the two cold plates 30, 32, with the side rails 54 slidably engageable with the frame receptacles R1, R2 between the cold plates 30, 32 and with the below-described membrane assembly passed through the slot 36 to be in close juxtaposition with the refrigerant channels in the cold plates.

In cross-references to Figures 4 and 5, the frame, in the example shown, the top rail 53 thereof, is formed with a fluid inlet 56 in which an inlet tube 58 has been disposed and a fluid outlet 60 in which an outlet tube 62 has been disposed. Both the inlet and outlet establish respective fluid passageways through the frame into the opening. The inlet and outlet tubes 58, 62 may be engaged with the fluid return and supply lines L3, L4 that are associated with the catheter 12. The tubes 58, 62 may terminate at just below the top rail 53 (Figure 4), or they may extend any desired length down to the bottom of the assembly, i.e., the tubes 58, 62 may extend almost the entire length of the left and right side rails 54, ending just above the below-described bottom seam of the membrane assembly.

Indeed, a polymeric membrane assembly 64 is connected to the frame 52, blocking the opening that is bounded on three sides by the frame as shown. The membrane assembly includes a first membrane 66 that is parallel to and closely spaced from a second membrane 68, leaving a space there between which establishes a working fluid chamber. The fluid inlet 56 and fluid outlet 60 communicate with the space between the membranes 66, 68. At least one and preferably both of the membranes 66, 68 are disposed in tension in the opening. The space between the membranes is expandable when filled with working fluid.

In one example, each membrane is no more than two mils (0.002") (0.051 mm) thick and more preferably is between one mil and two mils in thickness (0.001" - 0.002") (25.4 - 50.8 micrometres), inclusive. The example preferred membranes 66, 68 are coextensive with the opening and like the opening are more or less square, with the length of top and bottom edges of the example membranes being approximately equal (within ±10% and more preferably within ±5%) of the lengths of the left and right edges of the membranes. Thus, the working fluid chamber between the membranes is also rectilinear and in the preferred embodiment no obstructions exist between the membranes, meaning the working fluid chamber is a complete rectilinear, more or less square chamber.

Owing to the thinness of the membranes 66, 68 and the closeness of the cold plates 30, 32 to each other and to the membrane assembly between them when the cassette is engaged with the cold plates, the system shown in the figures affords low impedance of heat transfer between the refrigerant circulating in the cold plates and the working fluid circulating between the membranes 66, 68. The working fluid chamber between the membranes inflates due to backpressure generated by working fluid flow, eliminating or reducing the need for a moving mechanism in the cold plates. Moreover, the narrow slot 34 between the two cold plates provides better heat transfer by reducing the conductive path length between the cold plates and the working fluid. The frame allows for ease of handling, such as insertion and removal of the cassette with/from the cold plates.

With respect to the example working fluid chamber between the membranes 66, 68 having a width-to-length aspect ratio near 1:1 (i.e., square or nearly so), the amount of backpressure required to induce working fluid flow through heat exchanger is reduced compared to a less square configuration. This reduces the amount of work that a working fluid pump must perform, which is desirable for two reasons. One, since the pump may be disposable, lower performance requirements translate into a lower cost disposable and quieter system. For instance, peristaltic roller pumps offer quiet operation and a low-cost disposable element, but operate most efficiently when only modest pressures are required. Two, lowering the working fluid pump work reduces the amount of heat transferred into the working fluid by the pump itself. Also, a low width/length aspect ratio results in slower working fluid velocity which reduces amount of mixing, but this otherwise desirable (from a heat exchange standpoint) effect is negligible in the present example system since the Reynolds numbers are typically < 1000, suggesting a laminar flow regime. Furthermore, a low width/length aspect ratio significantly reduces the number of bends (or "corners') in the fluid flow path. These bends are areas of mixing for the fluid which promotes heat transfer. Without them, a fluid boundary layer builds up. However, this effect is offset herein by maintaining a narrow slot between the cold plates. This way the primary heat transfer mechanism is by conduction, but the conduction path length (and therefore boundary layer) is small, resulting in a relatively high rate of beat transfer.

In preferred examples, the membranes 66, 68 are stretched under tension during assembly to the frame. This tension can be maintained over the shelf life of the product. Pretensioning minimizes wrinkles in material, which is beneficial because wrinkles can impede working fluid flow and create air gaps which reduce heat transfer between the working fluid and cold plates. Wrinkles can also complicate insertion of the membrane assembly into the narrow slot 34.

To establish pre-tensioning of the membranes, the frame may be made in halves and posts such as threaded fasteners 70 (Figure 5) can extend transversely to one half of the frame, with the membranes 66, 68 being stretched over the posts and holes made in the membranes to receive the posts. The other half of the frame is then positioned to sandwich a rectilinear border portion 74 (only the innermost portion of which is shown in Figure 5) of the membrane assembly between the frame halves, and a closure such as respective nuts 72 engaged with the posts 70 to hold the frame halves together with the membrane assembly held in tension between the frame halves. Figure 4 shows that the working fluid chamber is closed off at the bottom by a bottom seam 74A of the membrane assembly, which is part of the border portion 74.

In the border portion 74, at least one and preferably more layers of polymer film may be used to reinforce the membranes 66, 68 to establish welded seams through which (at the sides of the membrane assembly) the post holes are formed, allowing for easier fabrication. By placing reinforcing layers on the border portion 74 only, the central "window" of the membrane assembly consists only of a single thin layer membrane 'between the working fluid and one of the cold plates 30, 32 to minimize impeding heat transfer. A die-cut reinforcement layer may be used which reinforces the entire perimeter with one piece of material.

In some examples, the polymer membranes 66, 68 are highly stretchable, at least greater than 25% elongation. This allows the membranes to change from the empty flat state shown in Figures 4 and 5 to an inflated shape (within the slot 34 between the cold plates) without wrinkling, it also allows the membranes to easily conform to features on the faces of the cold plates.

Additionally, the membranes may be made of a material which can also be made into tubing. Tubes such as the inlet and outlet tubes 58, 62 shown in Figure 4 can then be thermally welded (e.g., using RF sealing) to the membranes, which is more reliable and quicker than adhesive bonding. The membranes 66, 68 need not provide their own lateral support because the cold plates 32, 34 and frame provide the support for the inflated membrane assembly, allowing it to withstand the pressure generated as a result of working fluid flowing through between the membranes. Structural features may be located on the cold plates to optimize heat transfer. This can be economically advantageous because the cold plates are reusable components. Manifolds can be cut into the cold plates to even out the distribution of saline flow.

While the particular PATIENT HEAT EXCHANGE SYSTEM WITH TRANSPARENT WALL FOR VIEWING CIRCULATING REFRIGERANT is herein shown and described in detail, the scope of the present invention is to be limited by nothing other than the appended claims.

## Claims

1. A working fluid cassette comprising:
a frame (52) defining a periphery and an opening, the frame (52) being configured for being closely received between two cold plates (30, 32), the frame (52) having at least a fluid inlet (56) and a fluid outlet (60) both establishing respective fluid passageways through the frame (52) into the opening, the fluid inlet (56) and outlet (60) being configured for fluid communication with respective fluid return and supply lines (L1, L2, L3, L4) configured for coupling to a patient-engageable heat exchange member (12, 18), wherein the frame (52) comprises a first half and a second half;
a membrane assembly (64) connected to the frame (52) and blocking the opening, the membrane assembly (64) including a space, the fluid inlet (56) and fluid outlet (60) communicating with the space, the membrane assembly comprising a first membrane (66) and a second membrane (68) being closed on all sides; and
a plurality of posts (70) extending from the first half of the frame (52), wherein at least a portion of the membrane assembly (64) having holes is stretched over the posts (70),
wherein the second half of the frame is positioned to sandwich the portion of the membrane assembly (64) between the first and second halves of the frame (52), and
wherein a closure engages with the posts (70) to hold the frame halves together with the membrane assembly held in tension between the frame halves.

2. The working fluid cassette of Claim 1, wherein the space is expandable when filled with working fluid circulating from the heat exchange member (12, 18).

3. The working fluid cassette of Claim 1, wherein the opening defines a top, a bottom edge spaced from and parallel to the top, a left side extending between the top and bottom, and a right side extending between the top and bottom and parallel to the left side, the left and right sides defining a first length, the top and bottom defining a second length, the first length being equal to the second length ± ten percent of the second length.

4. The working fluid cassette of Claim 3, wherein the first length is equal to the second length.

5. The working fluid cassette of Claim 1, wherein the membrane assembly (64) defines a rectilinear border (74) juxtaposed with the frame (52), the working fluid cassette further comprising at least one reinforcing layer engaged with the first or second membrane at the rectilinear border (74).

6. The working fluid cassette of Claim 1, comprising at least one reinforcing layer, wherein the at least one reinforcing layer engages the membrane assembly in a border of the membrane assembly.

7. The working fluid cassette of Claim 6, wherein the at least one reinforcing layer engages the membrane assembly in the border of the membrane assembly that is disposed between the halves of the frame.

8. The working fluid cassette of Claim 1, wherein the fluid inlet (56) and fluid outlet (60) are defined at least in part by respective inlet and outlet tubes (58, 62).

9. The working fluid cassette of Claim 1 in which the membrane assembly comprises a membrane portion which defines the space as a working fluid chamber.

10. A system comprising:
the working fluid cassette of Claim 1; and
the two cold plates (30, 32).

11. The system of Claim 10, further comprising the patient-engageable heat exchange member (12, 18).

12. The system of Claim 11, wherein the patient-engageable heat exchange member comprises an intravascular heat exchange catheter (12) or a heat exchange pad (18) externally engageable with a patient.

## Patentansprüche

1. Arbeitsfluidkassette, umfassend:
einen Rahmen (52), der einen Umfang und eine Öffnung definiert, wobei der Rahmen (52) so konfiguriert ist, dass er eng zwischen zwei Kühlplatten (30, 32) empfangen wird, wobei der Rahmen (52) mindestens einen Fluideinlass (56) und einen Fluidauslass (60) aufweist, die beide entsprechende Fluiddurchgänge durch den Rahmen (52) in die Öffnung herstellen, wobei der Fluideinlass (56) und der Fluidauslass (60) für die Fluidkommunikation mit entsprechenden Fluidrückführungs- undversorgungsleitungen (L1, L2, L3, L4) konfiguriert sind, die zum Koppeln mit einem am Patienten eingreifenden Wärmetauscherelement (12, 18) konfiguriert sind, wobei der Rahmen (52) eine erste Hälfte und eine zweite Hälfte umfasst;
eine Membranbaugruppe (64), die mit dem Rahmen (52) verbunden ist und die Öffnung verschließt, wobei die Membranbaugruppe (64) einen Raum einschließt, wobei der Fluideinlass (56) und der Fluidauslass (60) mit dem Raum kommunizieren, wobei die Membranbaugruppe eine erste Membran (66) und eine zweite Membran (68) umfasst, die auf allen Seiten geschlossen sind; und
eine Vielzahl von Pfosten (70), die sich von der ersten Hälfte des Rahmens (52) aus erstrecken, wobei mindestens ein Abschnitt der Membranbaugruppe (64) mit Löchern über die Pfosten (70) gespannt ist,
wobei die zweite Hälfte des Rahmens so positioniert ist, dass sie den Abschnitt der Membranbaugruppe (64) zwischen der ersten und der zweiten Hälfte des Rahmens (52) einklemmt, und
wobei ein Verschluss in die Pfosten (70) eingreift, um die Rahmenhälften zusammenzuhalten, wobei die Membranbaugruppe zwischen den Rahmenhälften unter Spannung gehalten wird.

2. Arbeitsfluidkassette nach Anspruch 1, wobei der Raum erweiterbar ist, wenn er mit Arbeitsfluid gefüllt ist, das von dem Wärmetauscherelement (12, 18) zirkuliert.

3. Arbeitsfluidkassette nach Anspruch 1, wobei die Öffnung eine Oberseite, eine untere Kante, die von der Oberseite beabstandet ist und parallel zu dieser verläuft, eine linke Seite, die sich zwischen der Oberseite und der Unterseite erstreckt, und eine rechte Seite, die sich zwischen der Oberseite und der Unterseite und parallel zu der linken Seite erstreckt, definiert, wobei die linke und die rechte Seite eine erste Länge definieren, die Oberseite und die Unterseite eine zweite Länge definieren, wobei die erste Länge gleich der zweiten Länge ± zehn Prozent der zweiten Länge ist.

4. Arbeitsfluidkassette nach Anspruch 3, wobei die erste Länge gleich der zweiten Länge ist.

5. Arbeitsfluidkassette nach Anspruch 1, wobei die Membranbaugruppe (64) eine geradlinige Grenze (74) definiert, die an den Rahmen (52) angrenzt, und die Arbeitsfluidkassette ferner mindestens eine Verstärkungsschicht umfasst, die in die erste oder zweite Membran an der geradlinigen Grenze (74) eingreift.

6. Arbeitsfluidkassette nach Anspruch 1, umfassend mindestens eine verstärkende Schicht, wobei die mindestens eine verstärkende Schicht in die Membranbaugruppe an einer Grenze der Membranbaugruppe eingreift.

7. Arbeitsfluidkassette nach Anspruch 6, wobei die mindestens eine verstärkende Schicht in die Membranbaugruppe an der Grenze der Membranbaugruppe eingreift, die zwischen den Hälften des Rahmens angeordnet ist.

8. Arbeitsfluidkassette nach Anspruch 1, wobei der Fluideinlass (56) und der Fluidauslass (60) mindestens zum Teil durch entsprechende Einlass- und Auslassrohre (58, 62) definiert sind.

9. Arbeitsfluidkassette nach Anspruch 1, bei der die Membranbaugruppe einen Membranabschnitt umfasst, der den Raum als Arbeitsfluidkammer definiert.

10. System, umfassend:
die Arbeitsfluidkassette nach Anspruch 1; und
die beiden Kühlplatten (30, 32).

11. System nach Anspruch 10, ferner umfassend das in den Patienten eingreifende Wärmetauscherelement (12, 18).

12. System nach Anspruch 11, wobei das mit dem Patienten eingreifbare Wärmetauscherelement einen intravaskulären Wärmetauscherkatheter (12) oder ein Wärmetauschpad (18) umfasst, das von außen in den Patienten eingreifen kann.

## Revendications

1. Cassette de fluide de travail comprenant :
un cadre (52) définissant une périphérie et une ouverture, le cadre (52) étant conçu pour être reçu étroitement entre deux plaques froides (30, 32), le cadre (52) présentant au moins une entrée de fluide (56) et une sortie de fluide (60) établissant toutes deux des passages de fluide respectifs à travers le cadre (52) dans l'ouverture, l'entrée de fluide (56) et la sortie de fluide (60) étant conçues pour une communication fluidique avec des conduites respectives de retour et d'alimentation en fluide (L1, L2, L3, L4 ) conçues pour être couplées à un élément d'échange thermique pouvant être engagé par le patient (12, 18), dans laquelle le cadre (52) comprend une première moitié et une seconde moitié ;
un ensemble de membrane (64) relié au cadre (52) et bloquant l'ouverture, l'ensemble de membrane (64) comportant un espace, l'entrée de fluide (56) et la sortie de fluide (60) communiquant avec l'espace, l'ensemble de membrane comprenant une première membrane (66) et une seconde membrane (68) étant fermées de tous les côtés ; et
une pluralité de montants (70) s'étendant à partir de la première moitié du cadre (52), dans laquelle au moins une partie de l'ensemble de membrane (64) présentant des trous est tendue sur les montants (70),
dans laquelle la seconde moitié du cadre est positionnée pour prendre en sandwich la partie de l'ensemble de membrane (64) entre les première et seconde moitiés du cadre (52), et
dans lequel une fermeture vient en prise avec les montants (70) pour maintenir les moitiés de cadre ensemble, l'ensemble de membrane étant maintenu en tension entre les moitiés de cadre.

2. Cassette de fluide de travail selon la revendication 1, dans laquelle l'espace est extensible lorsqu'il est rempli de fluide de travail circulant depuis l'élément d'échange thermique (12, 18).

3. Cassette de fluide de travail selon la revendication 1, dans laquelle l'ouverture définit un bord supérieur, un bord inférieur espacé du haut et parallèle au haut, un côté gauche s'étendant entre le haut et le bas, et un côté droit s'étendant entre le haut et le bas et parallèle au côté gauche, les côtés gauche et droit définissant une première longueur, le haut et le bas définissant une seconde longueur, la première longueur étant égale à la seconde longueur ± dix pour cent de la seconde longueur.

4. Cassette de fluide de travail selon la revendication 3, dans laquelle la première longueur est égale à la seconde longueur.

5. Cassette de fluide de travail selon la revendication 1, dans laquelle l'ensemble de membrane (64) définit une bordure rectiligne (74) juxtaposée au cadre (52), la cassette de fluide de travail comprenant en outre au moins une couche de renforcement en prise avec la première ou la seconde membrane au niveau de la bordure rectiligne (74).

6. Cassette de fluide de travail selon la revendication 1, comprenant au moins une couche de renforcement, dans laquelle l'au moins une couche de renforcement vient en prise avec l'ensemble de membrane dans une bordure de l'ensemble de membrane.

7. Cassette de fluide de travail selon la revendication 6, dans laquelle l'au moins une couche de renforcement vient en prise avec l'ensemble de membrane dans la bordure de l'ensemble de membrane qui est agencée entre les moitiés du cadre.

8. Cassette de fluide de travail selon la revendication 1, dans laquelle l'entrée de fluide (56) et la sortie de fluide (60) sont définies au moins en partie par des tubes d'entrée et de sortie respectifs (58, 62).

9. Cassette de fluide de travail selon la revendication 1, dans laquelle l'ensemble de membrane comprend une partie de membrane qui définit l'espace comme chambre de fluide de travail.

10. Système comprenant :
la cassette de fluide de travail selon la revendication 1 ; et
les deux plaques froides (30, 32).

11. Système selon la revendication 10, comprenant en outre l'élément d'échange thermique pouvant être engagé par le patient (12, 18).

12. Système selon la revendication 11, dans lequel l'élément d'échange thermique pouvant être engagé par le patient comprend un cathéter d'échange thermique intravasculaire (12) ou un tampon d'échange thermique (18) pouvant s'engager extérieurement avec un patient.
